Europäisches Patentamt

European Patent Office

Office européen des brevets

(11) Publication number: **0 247 880**
**A2**

(12) **EUROPEAN PATENT APPLICATION**

(21) Application number: **87304744.3**

(22) Date of filing: **28.05.87**

(51) Int. Cl.4: **A 61 B 5/02**

(30) Priority: **28.05.86 US 868251**

(43) Date of publication of application:
**02.12.87 Bulletin 87/49**

(84) Designated Contracting States: **DE FR GB**

(71) Applicant: **BAXTER TRAVENOL LABORATORIES, INC.**
**One Baxter Parkway**
**Deerfield, IL 60015 (US)**

(72) Inventor: **Link, William Trevor**
**130 The Uplands**
**Berkeley California 94705 (US)**

(74) Representative: **Bizley, Richard Edward et al**
**BOULT, WADE & TENNANT 27 Furnival Street**
**London EC4A 1PQ (GB)**

(54) **An oscillometric blood pressure monitoring device.**

㊗ An assembly for use in oscillometric blood pressure monitoring, comprising:

(a) a tube (36) having a free end (34) and a flexible, stretchable bladder (24) which is mounted to and across the free end (34) of the tube (36) and which is pressurizable up to a pressure of at least about 200 Torr so as to expand out in front of the free end (34) of the tube (36) and means (42) for limiting the lateral expansion of the bladder (24) relative to the tube (36) in a way which will allow it to produce pressure pulses when the bladder (24) is placed externally directly against the person adjacent the artery (14) such that the bladder (24) and not the tube (36) engages the person and is thereafter pressurized in the controlled way.

(b) means (28) for pressurizing the bladder (24) through the tube (36) in the controlled way; and

(c) means (26) for holding the bladder (24) externally against the temporal artery (14) as the bladder (24) is pressurized in the controlled way.

EP 0 247 880 A2

**Description**

AN OSCILLOMETRIC BLOOD PRESSURE MONITORING DEVICE

The present invention relates generally to blood pressure monitoring techniques and more particularly to an oscillometric blood pressure monitoring assembly which is especially designed to take blood pressure in the vicinity of the temporal artery of a person.

Oscillometric techniques for obtaining various information about the blood pressure of a given person are well known in the art, as exemplified in United States Patents 3,903,872, 4,009,709 and 4,074,711 which are incorporated herein by reference. In all of these techniques, the blood pressure cuff, pad or like bladder type member are externally positioned against an artery of the subject being monitored and pressurized in a controlled manner so as to produce a series of pressure pulses which are responsive to an actual blood pressure within the cooperating artery. These pressure pulses can then be analyzed to obtain the desired information including, for example, diastolic and systolic pressures. In United States patent 3,903,872 a technique is described for determining the diastolic pressure of a subject by appropriately analyzing these pressure pulses. In United States Patents 4,009,209 and 4,074,711, techniques are described for determining systolic pressure. In all three of these patents, a conventional blood pressure cuff is utilized as a pressurizable pressure transducing bladder disposed around the arm or thigh of the subject.

The oscillometric measurement of blood pressure requires proper calibration between the pressure applied to the external surface of the body and the resultant pressure applied to an underlying target artery. The normal arrangement utilizes a pressure cuff wrapped entirely around the limb which contains the artery, as suggested in the patents recited above. This is because a pressure cuff can be made sufficiently long such that the applied pressure in the cuff is reflected in a nearly equal pressure being applied to the artery, at least near the center of the cuff. One advantage in using a blood pressure cuff around a limb of a subject being monitored is that the cuff is old technology and is relatively uncomplicated in design. One disadvantage to its use is that the limb tends to be susceptible to muscular artifacts. Another disadvantage resides in the relatively large fluid supply needed to pressurize the cuff. Typically, a pressurized adult arm cuff requires a fluid volume of about 500 cubic centimeters. To pressurize a volume of that magnitude to a pressure above the anticipated systolic pressure of a person, often a pressure of about 160 torr requires a relatively large pressure pump.

Still another disadvantage to using a pressure cuff around a limb arises when it is desirable or necessary to have reliable information regarding the blood pressure at the brain. The limbs are so far from the head that the information provided may not always be truly representative of the blood pressure at the brain.

As will be described in more detail hereinafter, the assembly of the present invention is not designed to be used with an artery contained within the subject's limb but rather that person's temporal artery, one of its branches, or any other suitable artery adjacent the subject's skull, hereinafter referred to merely as the temporal artery or temporal location. Accordingly, the present invention provides a bladder assembly for use in oscillometric blood pressure monitoring which, when placed externally against the temporal artery in a particular manner and pressurized in a controlled way, produces the necessary series of oscillometric pressure pulses that can be analyzed for the desired information. By using a temporal bladder rather than a limb cuff, the bladder can be placed at a location which is sufficiently close to the temporal artery to produce the desired pressure pulses but which is substantially free of any muscular movement, whereby to minimize the presence of muscular artifacts. Also, the temporal bladder can be made extremely small in size, for example no more than 1 to 2 cubic centimeters in volume, so that miniaturized sources of pressure can be used to pressurize the bladder.

Specifically, the assembly of the invention comprises an assembly for use in oscillometric blood pressure monitoring, comprising:

(a) a tube having a free end and a flexible, stretchable bladder which is mounted to and across the free end of the tube and which is pressurizable up to a pressure of at least about 200 Torr so as to expand out in front of the free end of the tube and means for limiting the lateral expansion of the bladder relative to the tube in a way which will allow it to produce pressure pulses when the bladder is placed externally directly against the person adjacent an artery such that the bladder and not the tube engages the person and is thereafter pressurized in the controlled way:

(b) means for pressurizing the bladder through the tube in the controlled way; and

(c) means for holding the bladder externally against the temporal artery as the bladder is pressurized in the controlled way.

Included within the invention is a tube having a free end and a flexible, stretchable bladder which is mounted to and across the free end of the tube and which is pressurizable up to a pressure of at least about 200 Torr so as to expand out in front of the free end of the tube and means for limiting the lateral expansion of the bladder relative to the tube in a way which will allow it to produce pressure pulses when the bladder is placed externally directly against the person adjacent an artery such that the bladder and not the tube engages the person and is thereafter pressurized in the controlled way.

The invention is hereinafter particularly described with reference to obtaining information about blood pressure in the temporal artery (including other arteries adjacent the skull) but the device may be used in relation to other arteries.

The system is further described by way of example hereinafter in conjunction with the drawings wherein:

Figure 1 diagrammatically illustrates a system for obtaining certain information about the blood pressure of a given person by means of oscillometry utilizing a temporal pressure transducing bladder assembly designed in accordance with one embodiment of the present invention;

Figure 2 is an enlarged diagrammatic illustration, partially in cross section, displaying part of the temporal pressure transducing bladder assembly shown in Figure 1;

Figure 3 is an enlarged diagrammatic illustration of a temporal pressure transducing bladder assembly designed in accordance with a further embodiment of the present invention;

Figure 4 is a view similar to Figure 3 but illustrating a temporal pressure transducing bladder assembly designed in accordance with still a further embodiment of the present invention; and

Figure 5 is a view similar to Figure 3 but illustrating an assembly designed in accordance with still a further embodiment of the present invention.

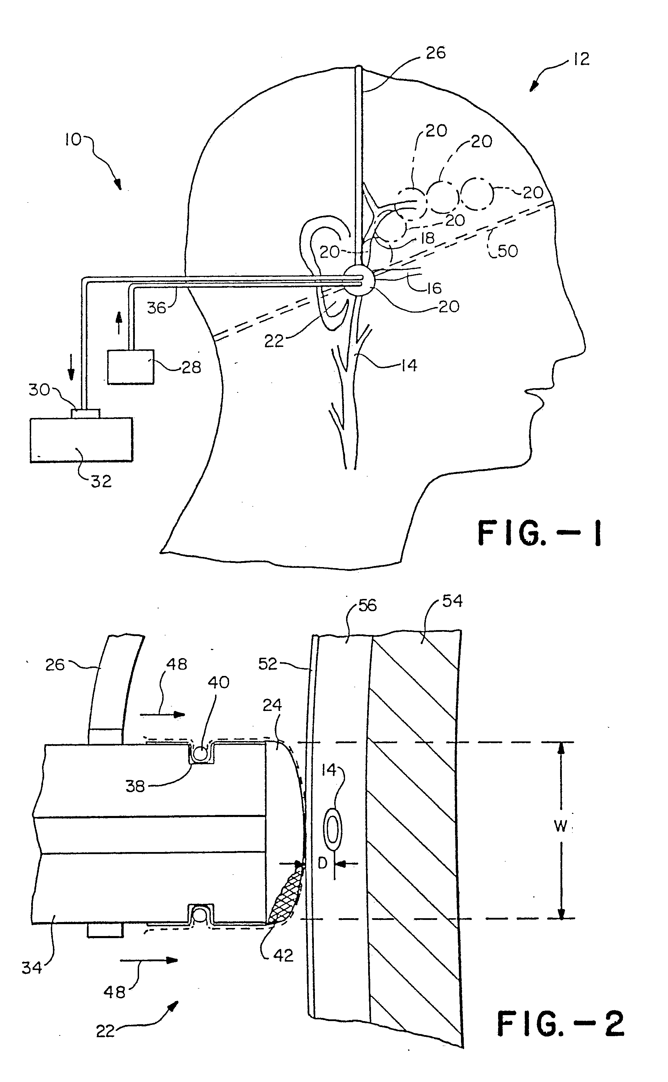

Turning now to the drawings, attention is immediately directed to Figure 1 which, as indicated above, discloses a system for obtaining certain information about the blood pressure of a given person. The system is generally indicated at 10 and the head of the person is shown at 12. The temporal artery 14 is also shown along with two of its orbital and parietal branches 16 and 18, respectively, located near the temple of individual 12. For purposes herein, the temporal artery and its branches and any other suitable artery adjacent the subject's skull will all be referred to merely as the temporal artery. Also, it should be noted that there are a number of temporal points 20 near where the branches 16 and 18 meet which include few if any muscles. Small scalp muscles attach above these points and the masseter muscles attach below them. As a result, even if individual 12 moves his jaw or his scalp, there is virtually no muscular movement at the points just described.

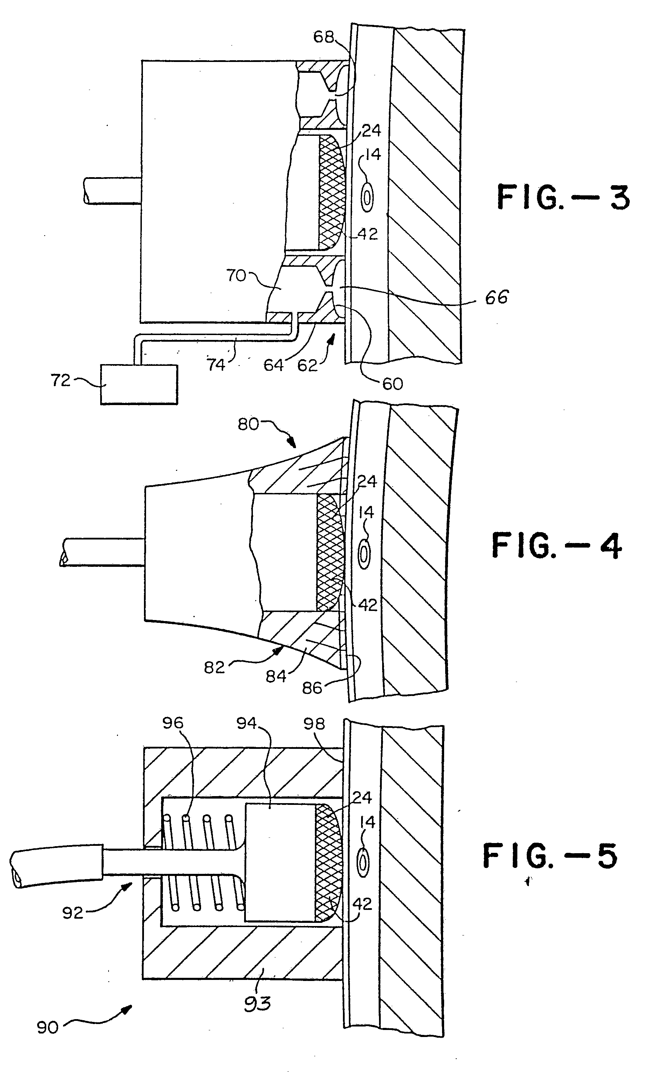

Still referring to Figure 1, overall system 10 includes a blood pressure transducing bladder assembly 22 sometimes referred to hereinafter as "cuff", which is designed in accordance with the present invention for use in cooperation with the temporal artery of individual 12. As will be seen below, this assembly includes its own pressurizable pressure transducing bladder 24 (see Figure 2) and means generally indicated at 26 for holding the bladder externally against the temporal artery of individual 12 in a particular way to be described below, preferably at points along the branches of the temporal artery.

System 10 also includes suitable means 28 for pressurizing the bladder in a controlled way for producing a series of pressure pulses that are responsive to the actual blood pressure within the cooperating temporal artery. These cuff pulses are converted to corresponding electronic signals by suitable transducer 30 and then these signals are acted upon by suitable circuitry 32 in order to extract therefrom the desired information about the blood pressure of individual 12. The particular way in which membrane 24 is pressurized to produce the desired pressure pulses is known in the art and will not be described herein. It suffices to say that the pressure within the membrane is ramped upward or downward between a minimum pressure of zero torr and a maximum which is slightly above the anticipated systolic pressure of the subject being evaluated, for example around 160 Torr. Also, both transducer 30 and circuitry 32 are known in the art and hence will not be discussed herein. Examples of suitable circuitry may be found in the above recited patents.

Referring specifically to Figure 2, attention is directed to pressure transducing bladder assembly 22 which is shown including one end section 34 of a tube or tube means 36 which is connected at its other end with pressurizing means 28, as shown in Figure 1. Tubular end section 34 includes an outer annular groove 38 for reasons to become apparent. The previously recited bladder or membrane 24 is positioned over the end of tubular end segment 34 including groove 38 and sealed about the circumference of groove 38 by means of an elastic sealing ring 40 or the like. The bladder is both flexible and stretchable in an actual working embodiment and is formed from rubber about 2 mils thick or thicker. Thus, air or other suitable fluid under pressure is supplied from means 28 into tubular means 36, which causes bladder 24 to expand. However, the overall bladder assembly includes means in the form of flexible but unstretchable netting 42 for limiting the stretchability of the bladder in a predetermined way which will be discussed below. The netting itself may be integrally formed within bladder 24 as reinforcing fibers or, as illustrated in Figure 2, it may be located directly over the bladder and under sealing ring 40.

Bladder 24 and its cooperating netting 42 are held externally against the temporal artery 14 in the manner shown in Figure 2 by means 26 which, as illustrated in Figure 1, is in the form of a head clamp designed to urge the bladder in the direction of the temporal artery, as diagrammatically depicted by means of arrows 48. In order to get the appropriate cooperation between bladder 42 and the temporal artery 14 to produce accurate pressure pulses, it is important that a relatively wide, continuous segment of the bladder remain in contact with the outer skin of individual 12 over suitable branches of the temporal artery throughout the pressurizing cycle of the bladder. Thus, the clamp 26 or, alternatively, a head band 50 shown by dotted lines in Figure 1 must supply sufficient force 48 to prevent the bladder from pushing away from temporal point 20 as it is pressurized to its maximum level. At the same time, membrane 24 cannot be allowed to stretch laterally outward from the end tubular segment 34. It is prevented from doing so by netting 42.

With these conditions in mind, it can be seen that bladder 24 cooperates with temporal artery 14 in a manner which is similar to the way in which a pressure cuff around a particular limb cooperates with the artery contained by that limb. Specifically, as illustrated in Figure 2, temporal artery 14 is quite close to the skin for the temporal or any branch of the temporal artery and immediately in front of the underlying skull bone which provides a backing pressure. The temporal skin is indicated at 52, the skull bone is shown at 54 and the temporal artery 14 is therebetween in tissue 56. Note that the width W of the bladder is substantially greater than the depth D to the artery. Under these circumstances, the bladder pressure will be reflected in a nearly equal pressure being applied to the artery, at least near the center of the bladder in the same manner as exists between a blood pressure cuff and its cooperating limb artery.

In view of the foregoing, it should be apparent that by placing bladder 24 at temporal points 20, muscular artifacts are reduced and a more accurate picture of the blood pressure of the external carotid and therefore of the brain can be obtained than taking the pressure in a limb. However, another advantage to temporal pressure transducing bladder assembly 22 is that the bladder can be made to be extremely small and still operate in the intended manner. Specifically, when the chamber defined by the bladder is in its fully stretched condition (as limited by netting 42) can be as small as 1-2 cubic centimeters in volume or smaller. This means that relatively small and even miniature pressure sources can be used to pressurize the bladder to its maximum contemplated pressure which is around 250 Torr. An example of a miniature pressure supply is a Piezoelectric pump. Another small supply of pressure is a $CO_2$ canister. It is even possible with such a small volume to use a source of heat to heat up a confined supply of gas in order to cause the latter to expand into and pressurize the bladder. These are only examples of different types of gas pressure supplies. Certainly, standard readily available pressure supplies presently being used in blood pressure devices readily serve as supply 28, although needlessly large.

Turning now to Figure 3, attention is directed to a pressure transducing bladder assembly 60 which is designed in accordance with a second embodiment of the present invention. Assembly 60 functions to cooperation with temporal artery 14 to produce pressure pulses in the same manner as assembly 22. In addition, assembly 60 may include the identical membrane 24, tubular end segment 34, sealing ring 40 and netting 42. However, assembly 60 includes entirely different means for holding bladder 24 against the temporal artery of individual 12. Specifically, assembly 60 includes a vacuum suction arrangement 62 which may take any suitable form. As illustrated in Figure 3, this arrangement is in the form of a concentric outer housing 64 defining a forwardly facing, circumferential suction cup 66 surrounding bladder 24. A plurality of passages 68 circumferentially spaced around the back end of the suction cup open into a common plenum 70 formed within housing 64. Plenum 70 is, in turn, connected with a vacuum pump or the like 72 through a suitable conduit 74. By maintaining a vacuum within plenum 70, the suction cup 66 can be used to hold bladder 24 in its operating position at temporal point 20.

Figure 4 illustrates still another embodiment of a pressure transducing bladder assembly which is generally indicated by the reference numeral 80. This assembly, like assembly 60, may include an identical bladder 24, tubular end segment 34, and netting 42. However, assembly 80 includes an entirely different means of holding membrane 24 against temporal artery 14. This arrangement which is generally indicated at 82 is an adhesive bonding arrangement including a plurality of splined sections 84 which entirely circumscribe bladder 24 to present a generally planar surface but one which can readily conform to a curved area such as the area around temporal points 20. These splined sections either carry individual adhesive segments 86, as shown, or a single continuous, annular section of adhesive may be applied to all of the splined sections as long as it is sufficiently flexible to conform to intended known planar surfaces.

Figure 5 illustrates still another embodiment of a pressure transducing bladder assembly generally indicated at 90. This assembly may include an identical bladder 24 supported by a tubular arrangement 92 which is partially located within a surrounding housing 93.

The bladder and an end segment 94 of the tubular arrangement are spring loaded in the forward direction against the subject's temple by means of a relatively weak compression spring 96 while means generally indicated at 98 holds the entire assembly against temporal artery 14. Means 98 can be any of the type described above.

## Claims

1. An assembly (22) for use in oscillometric blood pressure monitoring, comprising:

(a) a tube (36) having a free end (34) and a flexible, stretchable bladder (Z4) which is mounted to and across the free end (34) of the tube (36) and which is pressurizable up to a pressure of at least about 200 Torr so as to expand out in front of the free end (34) of the tube (36) and means (42) for limiting the lateral expansion of the bladder (24) relative to the tube (36) in a way which will allow it to produce pressure pulses when the bladder (24) is placed externally directly against the person adjacent an artery (14) such that the bladder (24) and not the tube (36) engages the person and is thereafter pressurized in the controlled way.

(b) means (28) for pressurizing the bladder (24) through the tube (36) in the controlled way; and

(c) means (26) for holding the bladder (24) externally against the temporal artery (14) as the bladder (24) is pressurized in the controlled way.

2. An assembly as claimed in claim 1 wherein a flexible membrane (24) serves as the bladder and the expansion limiting means includes netting (42) cooperating with the membrane (24) so as to limit the expansion of the membrane (24) to a predetermined configuration.

3. An assembly as claimed in claim 2 wherein the netting (42) is integrally formed into the membrane (24).

4. An assembly as claimed in claim 2 wherein the netting (42) is formed on the outside of the membrane (24).

5. An assembly as claimed in any one of the preceding claims, which is adapted to measure the pressure in the temporal artery or another artery adjacent the person's skull and wherein the bladder holding means comprises:

(a) head band means (50) configured to fit tightly about the head of the person;

(b) clamp means (26) configured to fit tightly about the head of the person;

(c) a vacuum suction arrangement (62); or

(d) an adhesive bonding arrangement (84,86).

6. An assembly as claimed in claim 5 (d) wherein the bonding arrangement includes a plurality of individual adhesive bonding segments (84) movable with respect to one another to a limited extent so as to conform to a non-planar surface such as the external temporal area of an individual.

7. An assembly as claimed in any one of the preceding claims wherein the expansion limiting means (42) limits the size of the bladder to an internal volume of at most about two cubic centimeters.

8. An assembly as claimed in any one of the preceding claims wherein the pressurizing means includes a piezoelectric pump, a source of $CO_2$ or means for heating gas.

9. An assembly as claimed in any one of the preceding claims which is connected to a transducer (30).

10. A bladder unit, comprising a tube (36) having a free end (34) and a flexible, stretchable bladder (24) which is mounted to and across the free end (34) of the tube (36) and which is pressurizable up to a pressure of at least about 200 Torr so as to expand out in front of the free end (34) of the tube (36) and means (42) for limiting the lateral expansion of the bladder (24) relative to the tube (36) in a way which will allow it to produce pressure pulses when the bladder (24) is placed externally directly against the person adjacent an artery (14) such that the bladder (24) and not the tube (36) engages the person and is thereafter pressurized in the controlled way, the bladder unit optionally being as further defined in any one or more of claims 2 to 4 or 7.

0247880

FIG. — 1

FIG. — 2

0247880

FIG.—3

FIG.—4

FIG.—5